# EUROPEAN PATENT APPLICATION

(11) **EP 3 799 824 A1**
(43) Date of publication of application: **07.04.2021**
(21) Application number: 19910861.4
(22) Date of filing: 19.11.2019
(51) Int. Cl.: A61C 7/00

(54) **METHOD OF PLACING AN ORTHODONTIC APPARATUS, TEMPLATE AND ORTHODONTIC APPARATUS**

(30) Priority: 17.01.2019 RU 2019101228
(71) Applicant: Obshhestvo s Ogranichennoj Onost tvetstven`yu «Ortosmajl», Moscow 119071 (RU)
(72) Inventor: BORZOV, Sergej Viktorovich, Moscow, 107241 (RU)
(74) Representative: AOMB Polska Sp. z.o.o.
(86) International application number: PCT/RU2019/050219
(87) International publication number: WO 2020/149768

(57) **Abstract**

The group of inventions herein relates to medicine, namely to orthodontic dentistry, and is intended for use in the holistic treatment of patients with anomalies of the dentoalveolar system using orthodontic micro-implants, which are micro-screws, mini screws, as well as screws for temporary cortical support.

The source data required for manufacture is as follows: a computer tomogram, cast teeth models, jaw model, and the size of the micro-implants to be installed. Using cast teeth models, plaster dental models are casted and scanned in order to obtain a 3D printed model of the patient's jaw.

Then the computer tomogram and 3D model of the patient's jaw are combined. Based on the anatomy and shape of the palatal mucosa, the doctor selects the entry points of the micro-implants into the mucosa on a 3D model of the patient's jaw. The position of the micro-implants at these selected points is plotted, taking into account their inclination and depth of insertion.

The correctness of the selected position of the micro-implants is checked using the computer tomogram. On the basis of data obtained, a caliper is modeled for inserting micro-implants for the subsequent installation of an orthodontic apparatus. The inventions make it possible to reduce the number of patient visits to the doctor by increasing the accuracy and direction of the micro-implant's insertion for subsequent orthodontic apparatus installation.

## Description

### TECHNICAL FIELD

The invention relates to medicine, specifically to orthodontic dentistry, and can be used to install orthodontic apparatus with orthodontic micro-implants, such as micro-screws, mini-screws, screws for temporary cortical support, in the complex treatment of patients with anomalies of the dentoalveolar system.

### BACKGROUND ART

In the comprehensive treatment of patients with dentoalveolar anomalies, orthodontic micro-implants, screws that are no longer than 14 mm in length, are increasingly being used. In orthodontics, micro-implants serve as supports on which the orthodontic apparatus, for example, is mounted. This raises the question of the most rational and safe method of inserting micro-implants followed by an orthodontic apparatus.

There is a known surgical polymeric caliper, which is a mouthpiece structure made of acrylic plastic with a thickness of about 5 mm, in the structure of which mandrins are placed for directional insertion of cylindrical dental implants (Dental Rehabilitation with Dental Implants. A clinical guide. / N. Zitzman and P. Scherer, eds. Lomakina. - M.: Azbuka Publishing house, 2005. 26 pages).

The disadvantage of this method is that the polymeric surgical caliper does not allow planning the insertion of micro-implants from the vestibular surface of the alveolar processes of the jaws, and between the diagnostic and surgical stages it is necessary to use a mandrin, or to make a new caliper design.

Also, there is a known method of placing an orthodontic micro-implant and a conductor used for its implementation, described in the patent RU No. 2332186. The method includes radiography, selection of the point, direction and depth of the micro-implant installation based on its results. Preliminary installation of a mandrin having a horizontally located binder and a tube sleeve located at one of its ends, and installation of an orthodontic micro-implant through it. Radiography is performed by using computed tomography.

The mandrin is assembled in the patient's mouth. For this purpose, a metric rod is pre-made from disposable material, the marks on which are set according to the computed tomography marks, a horizontal rod whose length is determined according to the computed tomography data, and a drill guide. All parts of the mandrin are fixed using a clear, quick-setting and viscous silicone mass.

The disadvantages of the described method are the difficulty in fixing the components of the mandrin in the patient's mouth with a transparent, quick-setting viscous silicone mass. As a result, the accuracy of positioning of the drill guide for micro-implant insertion in the selected location is reduced. In addition, the high labor intensity of the mandrin assembly technique with its simultaneous single use at each stage of micro-implant insertion increases the cost of the operation.

There is also a well-known method described in the Russian patent No. 2470609 Method of Placing an Orthodontic Micro-Implant and a Caliper for Its Implementation, Publ. December 27, 2012, priority date: March 9, 2011, selected as a prototype.

To implement the method, a working plaster model of the patient's jaw is obtained, using which the mandrin caliper is made in the form of a teeth protector of an elastic material repeating the shape of the surfaces of the vestibular dentition, alveolar process and hard palate. The caliper is obtained by thermoforming on Mini Star or Easy-Vac machines from 0.75 or 1 mm polymeric blanks (Ideal Clear, GAC-Dentsply). In the process of stamping a metric radiopaque grid of metal thread with a diameter of 0.3 mm with a coordinate cell side of 5 mm is pressed into the blank in the projection of the micro-implant insertion zone.

The made caliper is fixed in the oral cavity and the hard palate is radiographed using contact near-focus irradiation or visiography for diagnosis and selection of placement and surgical intervention at the next stage. A radiopaque grid on an elastic gauze allows precise identification of the insertion sites of orthodontic micro-implants.

After determining the size of the micro-implants and their insertion sites, fixing holes are made in the caliper in the selected location according to the shape and size of the micro-implants.

The disadvantage of the proposed method is that it is not possible to insert the micro-implants into the bone tissue at the required angle for the subsequent installation of, for example, an orthodontic apparatus on them. The method proposed in the patent allows to determine quite accurately only the place of micro-implants installation. Another disadvantage is the use of 0.75- or 1-mm polymer blanks for making calipers, the thickness of which is insufficient to set the direction of the micro-implant insertion. All of the above leads to the necessity to produce an orthodontic apparatus only after the direction of the axes of the inserted micro-implants has been determined. As a consequence, the total minimum number of patient's visits to a doctor when using the method proposed in the invention is at least three.

### SUMMARY OF INVENTION

The objective of the present invention is to develop a method for increasing the accuracy and direction of micro-implants insertion for subsequent orthodontic apparatus installation.

The technical result obtained as a result of applying the method proposed in the invention is to reduce the number of patient's visits to the doctor.
This problem is solved, and the technical result is achieved by performing a computer tomography of the patient. A model of the patient's jaw is created. A caliper for the introduction of micro-implants and an orthodontic apparatus are made.

The micro-implants are inserted according to the caliper, on which the orthodontic apparatus is installed. A virtual 3D model of the patient's jaw is created using the model of the patient's jaw. The computer program combines the computer tomogram and 3D model of the patient's jaw. The coordinates of micro-implants entry points into the mucosa surface are determined and the positions of micro-implants insertion axes in these points are plotted. Based on the determined coordinates of the micro-implant's entry points and the position of the micro-implant's insertion axes, a caliper with guide holes for micro-implants insertion and an orthodontic apparatus with mounting holes for fixing the orthodontic apparatus on the micro-implants are made. The made caliper is fixed in the patient's oral cavity. The caliper is inserted along the fixing holes of the caliper micro-implants. The caliper is removed. The orthodontic apparatus is placed on the inserted micro-implants.

The technical result is also achieved by the fact that the caliper for the insertion of micro-implants to install an orthodontic apparatus is made in the form of a mouth guard.

The caliper has fixing holes for micro-implants insertion. The coordinates of the entry points of the micro-implants into the mucosa surface and the position of the axis of the micro-implants in these entry points are determined using a computer program based on the combined computer tomogram and a 3D model of the patient's jaw.

The thickness of the caliper at the insertion site of the implants should be at least sufficient to maintain the position of the implant insertion axis.

Preferably, the caliper for the insertion of micro-implants for placement of orthodontic apparatus is provided with inspection holes.

The caliper for the insertion of micro-implants for the placement of an orthodontic apparatus can be made, for example, from photopolymer by 3D printing.

The technical result is also achieved by the fact that the orthodontic apparatus contains the main supporting and holding devices, auxiliary fixing elements, regulating parts and mounting holes. The relative positioning and axes of the mounting holes of the orthodontic apparatus are made based on the coordinates of micro-implants entry points into the mucosa surface and the position of micro-implants introduction axes in these entry points using a computer program based on the combined computer tomogram and a 3D model of the patient's jaw.

The orthodontic apparatus can be fabricated by 3D metal printing using the obtained computer model of the apparatus.
In another embodiment, the orthodontic apparatus may be manufactured by casting metal in a mold made from a burnable photopolymer model obtained on a 3D printer from a computer model of the apparatus.

Processing the patient's tomogram, 3D model of the patient's jaw and the selected size of micro-implants in the computer program allows to choose the coordinates of entry points of micro-implants into the mucosa surface and build the positions of micro-implants in these points, taking into account their inclination and depth of insertion in the absence of the patient. Based on the obtained data, the caliper for micro-implants and orthodontic apparatus insertion is made in the absence of the patient. The coordinates of micro-implants insertion points and micro-implants axis positions are selected in the computer program simultaneously with the mutual positioning and axes of orthodontic apparatus insertion holes, which allows exact coincidence of micro-implants position and micro-implants insertion axes with the insertion holes of the produced orthodontic apparatus. This coincidence allows easy and guaranteed placement of the orthodontic apparatus on the inserted micro-implants.

The caliper for micro-implants insertion for orthodontic apparatus installation is made as a mouthpiece with fixing holes for micro-implants insertion according to the 3D model of the patient's jaw. The placement on the mucosal surface and the direction of the axes of the fixing holes are carried out based on the selection of entry points of micro-implants into the mucosal surface and the construction of the position of micro-implants in these points, taking into account their inclination and depth of insertion. This makes it possible to select and guarantee an optimal and safe position of the inserted micro-implants for further installation of an orthodontic apparatus on them.

The thickness of the caliper at the place of implant insertion must be at least sufficient to maintain the position of the implant insertion axes, which guarantees coincidence of the coordinates and directions of the axes of the orthodontic apparatus insertion holes and the inserted micro-implants.

The micro-implant insertion caliper for orthodontic apparatus insertion can have viewing holes for convenience. The viewing holes allow to control the procedure of micro-implants insertion.

The caliper for the insertion of micro-implants for orthodontic apparatus installation can be made of photopolymer by 3D printing, which also allows to increase the accuracy and ensure the planned direction of micro-implants insertion.

The orthodontic apparatus is made with insertion holes, main support-retaining devices, auxiliary fixation elements and regulating parts. Thanks to the use of a computer program, the mutual positioning and axes of the orthodontic apparatus mounting holes are made based on the selection of entry points of micro-implants into the mucosa surface and the positioning of micro-implants in these points taking into account their inclination and insertion depth. Such a solution makes it possible to guarantee the coincidence of the position of the insertion holes of the orthodontic apparatus and the position of the inserted micro-implants as well as the coincidence of the axes of the inserted micro-implants and the insertion holes of the orthodontic apparatus.

To obtain high quality, the orthodontic apparatus is preferably made by 3D metal printing according to the obtained computer model of the apparatus, or by casting metal in a mold made according to the burnable photopolymer model obtained on a 3D printer according to the computer model of the apparatus.

### BRIEF DESCRIPTION OF DRAWINGS

The invention is further explained by a detailed description of a specific, but not limiting, example of the solution and the accompanying drawings, where:
Fig. 1 shows examples of input data for implementing the proposed method;
Fig. 2 shows a combined computer tomogram and 3D models of the patient's jaw;
Fig. 3 shows selected entry points of micro-implants into the mucosa on 3D model of the patient's jaw;
Fig. 4 shows a verification of the selected location of the micro-implants;
Fig. 5 shows a modeling of the apparatus abutments and fixing holes of the caliper;
Fig. 6 shows a modeling of the caliper shell;
Fig. 7 shows a combined shell and fixing holes of the caliper;
Fig. 8 shows ready viewing holes of the caliper;
Fig. 9 shows modeled individual elements of the orthodontic apparatus (ring and beam);
Fig. 10 shows an added standard element of orthodontic apparatus, a moving screw;
Fig. 11 shows combined functional and technological elements of the orthodontic apparatus.
Fig. 12 shows an example of a ready-to-use orthodontic apparatus.

### EMBODIMENT OF INVENTION

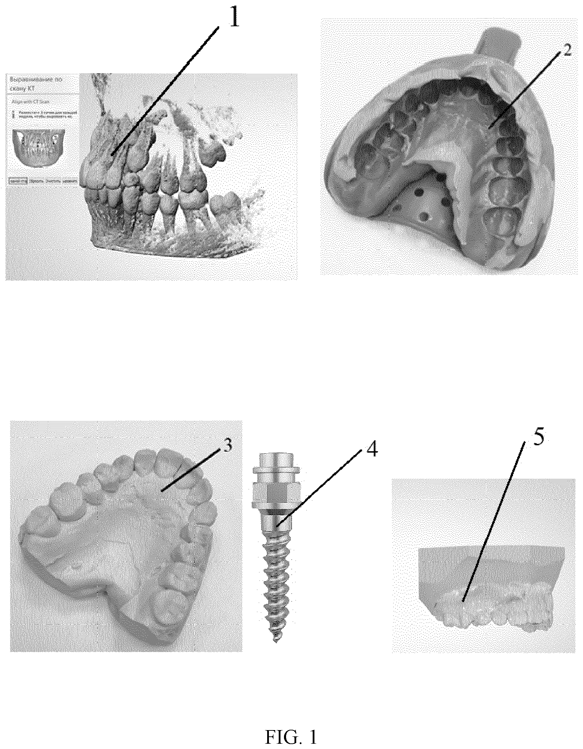

To implement the proposed method, the initial data are obtained, namely, a computer tomogram 1, dental cast model 2, plaster model 3 of the jaw, and select the size of the installed micro-implants 4. Based on impressions 2, plaster models 3, for example, are casted and scanned to obtain a 3D model 5 of the patient's jaw, as shown in Fig. 1.

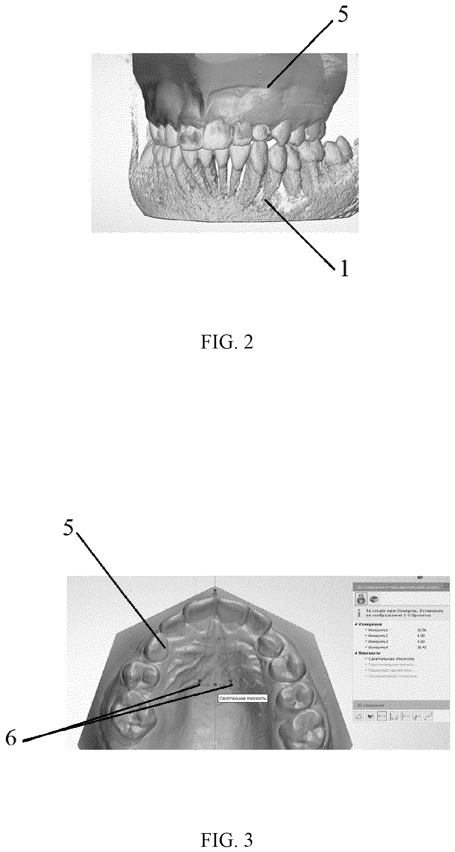

In the computer program, the computer tomogram 1 and 3D model 5 of the patient's jaw are combined, see Fig. 2.

Based on the anatomy and shape of the palatal mucosa, the entry points 6 of the micro-implants 4 into the mucosa are selected on the 3D model 5 of the patient's jaw, see Fig. 3.

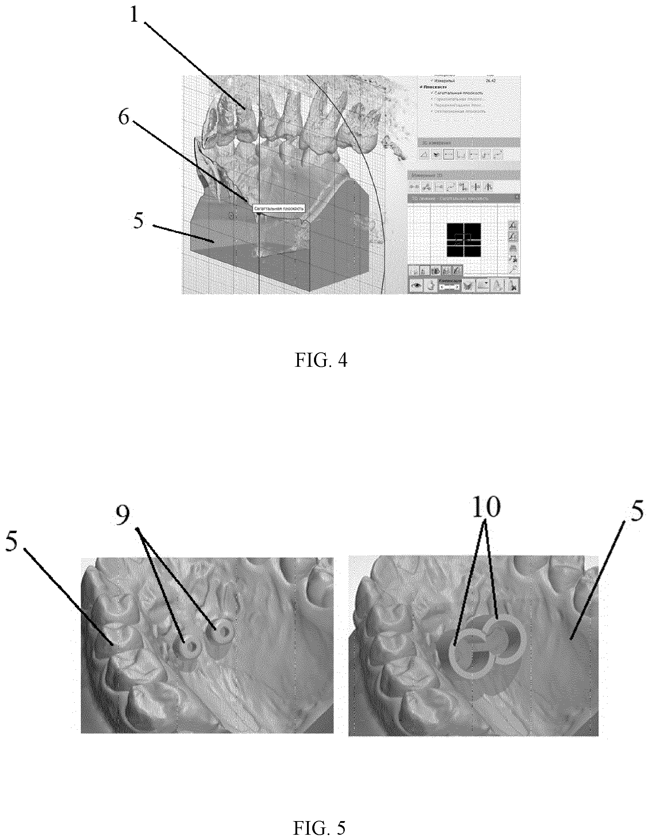

The position of micro-implants 4 in the given selected points 6 is plotted, taking into account their inclination and insertion depth. The correctness of the selected position of micro-implants 4 is checked on the computer tomogram, see Fig. 4.

If necessary, the positions of micro-implants 4 are corrected by repeating the selection of entry points 6 of micro-implants 4 into the mucosa on the 3D model 5 of the jaw and the selection of positions of micro-implants 4 in the given selected points 6 taking into account their inclination and depth of insertion.

Based on the data obtained, namely a 3D model 5 of the patient's jaw and a given position of the micro-implants 4 relative to it, a template 7 for introducing the micro-implants 4 is simulated for the subsequent installation of the orthodontic apparatus 8.

Using a computer program, the elements of the orthodontic apparatus 8 and the caliper 7 for introducing the micro-implants 4 directly associated with the micro-implants 4, such as the abutments 9 of the orthodontic apparatus 8, the fixing holes 10 of the caliper 7, are virtually set according to their position on the jaw, as shown in Fig. 5.

Thus, consistency of the bases of the orthodontic apparatus 8 and the caliper 7 for introducing the micro-implants 4 is achieved, and as a result, the positioning holes of the abutments 9 of the orthodontic apparatus 8 and the position of the introduced micro-implants 4, and the direction of the axes of the inserted micro-implants 4 and the positioning holes of the abutments 9 of the orthodontic apparatus 8 coincide.

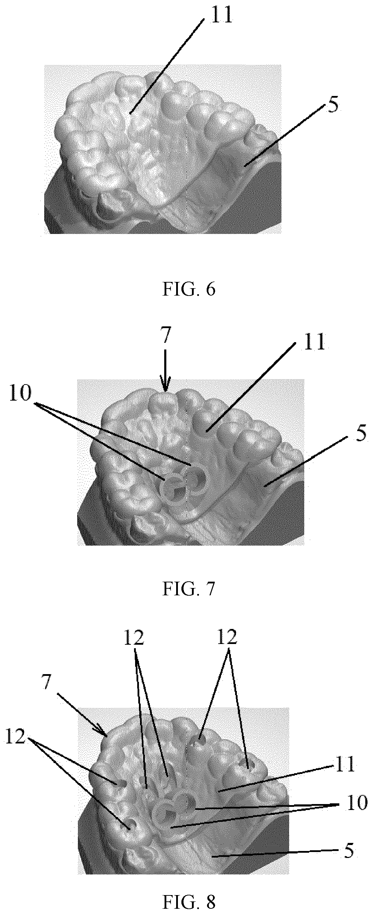

Creation of the caliper model 7 for the introduction of micro-implants 4 is a process of computer modeling according to the 3D model 5 of the jaw mouthpiece 11, adjacent to the palatal, occlusal and vestibular tooth surfaces and partially to the palatal mucosa, see Fig. 6. The mouthpiece 11 is combined in the computer program with the already created guide holes 10 for the insertion of the micro-implants 4, see Fig. 7. Then, observation holes 12 are placed in the mouthpiece 11 by means of the computer program to facilitate controlling the placement of the template 7 on the patient's jaw and observing the process of screwing in the micro-implants 4, see Fig. 8. Then, the caliper 7 created in the computer program for the introduction of the micro-implants 4 is made of photopolymer by 3D printing, while the thickness of the caliper 7 at the location of the guide holes 10 for the introduction of the micro-implants 4 must be sufficient to maintain the position of the micro-implant insertion axis during installation.

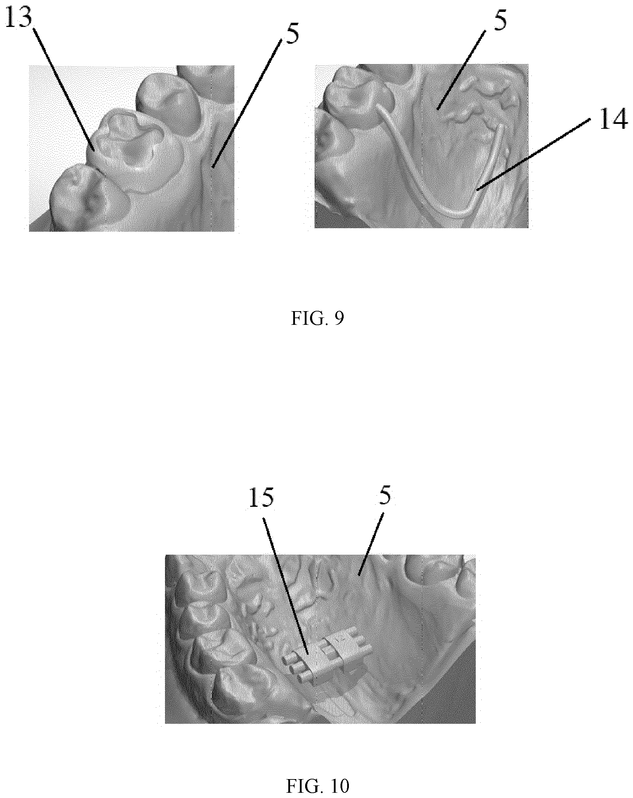

To create a model of the orthodontic apparatus 8, computer modeling of its individual elements, such as rings 13, beams 14, etc., is performed, as shown in Fig. 9. Also, if necessary, standard elements of the orthodontic apparatus moving screws 15, tube locks (not shown in the drawings), etc., are added, see Fig. 10. All elements are created and added in accordance with the required functions of the designed orthodontic apparatus 8, the 3D model 5 of the jaw and the position of the inserted micro-implants 4.

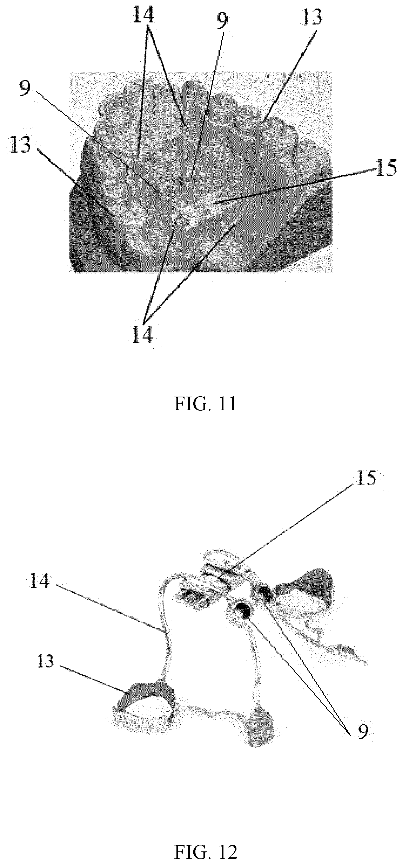

If necessary, the technological elements used to connect the parts of the orthodontic apparatus 8 are modeled so that it represents a monolithic structure during fabrication. Then all elements of the orthodontic apparatus 8 to be fabricated are combined in the computer program into a single body, see Fig. 11.

### INDUSTRIAL APPLICABILITY

Several technologies can be used to manufacture an orthodontic apparatus 8.

Alternatively, the fabrication can be done by 3D metal printing based on the obtained 3D computer model of the apparatus.

Optionally, it is possible to use casting, for which there is a need to do the following:
- Print the burnout model from photopolymer on a 3D printer;
- Form a gating system and make molds for casting;
- Cast metal into the resulting mold.

At the final stage, the moving screws 15 are welded and additional support elements made of dental plastic are manufactured.

An example of a manufactured orthodontic apparatus according to the proposed method is shown in Fig. 12.

Thus, the advantage of the proposed invention lies in the fact that the caliper 7 for the insertion of micro-implants 4 and the orthodontic apparatus 8 mounted on the introduced micro-implants 4 are made according to the computer 3D models, which allows to guarantee accuracy and to provide a planned direction of the introduction of micro-implants 4 for the subsequent installation of the orthodontic apparatus 8 on them. This, in turn, allows reducing the number of patient visits to the doctor.

The reduction in the frequency of patient visits to the doctor when using the proposed method has a positive effect on the emotional state of the patient.

It should be noted that the correction of anomalies of the dentoalveolar system is most often performed on children, and the implementation of the proposed method will reduce the emotional burden on children.

## Claims

1. The method of orthodontic ready-to-use apparatus placement is as follows: first, a computer tomography of the patient is performed, then a 3D model of the patient's jaw is created; then, a caliper for the insertion of micro-implants and orthodontic apparatus is made, the micro-implants are inserted according to the caliper, on which the orthodontic apparatus is installed, and the orthodontic apparatus is different in that the virtual 3D model of the patient's jaw is created from the jaw model; the computer program combines the computer tomogram and 3D model of the patient's jaw, and determines the coordinates of the entry points of the micro-implants into the mucous surface; the position of the micro-implant insertion axes in these points is plotted; based on the determined coordinates of the micro-implants' entry points and the position of the micro-implants' insertion axes, a caliper with fixing holes for micro-implants insertion and an orthodontic apparatus with mounting holes for fixing the orthodontic apparatus on the micro-implants are made; the caliper is fixed in the patient's mouth, the micro-implants are inserted through the fixing holes of the caliper, and the caliper is removed and the orthodontic apparatus is placed on the inserted micro-implants.

2. A caliper for the insertion of micro-implants to install an orthodontic apparatus, made in the form of a mouthpiece, differing in that it has fixing holes for the insertion of micro-implants, and determining the coordinates of entry points of micro-implants in the mucous surface and the construction of the axis of the micro-implants in these entry points is performed using a computer program by combining a computer tomogram and 3D-model of the jaw of the patient.

3. The caliper for inserting micro-implants for installation of an orthodontic apparatus according to claim 2 herein differing in that the thickness of the caliper at the place of insertion of micro-implants is sufficient to preserve the position of the axis of insertion of micro-implants.

4. The caliper for inserting micro-implants to install an orthodontic apparatus according to claim 2 herein differing in that it has observation holes.

5. The caliper for inserting micro-implants for an orthodontic apparatus according to claim 2 herein differs in that it is made of photopolymer by 3D printing.

6. An orthodontic apparatus containing main supporting and holding devices, auxiliary fixing elements, regulating parts and mounting holes, **characterized by** the fact that the mutual location and axes of the mounting holes of the orthodontic apparatus are made based on the coordinates of micro-implants entry points into the mucosa surface and the position of the micro-implants insertion axes in these entry points using the computer program on the combined computer tomogram and 3D-model of the patient's jaw.

7. The orthodontic apparatus similar to claim 4 herein, differing in that it is made by 3D printing with metal according to the obtained computer model of the apparatus.

8. The apparatus similar to claim 4 herein, differing in that it is made by casting metal in a mold made according to a burn-out photopolymer model obtained on a 3D printer from a computer model of the apparatus.
